# EUROPEAN PATENT SPECIFICATION

(11) **EP 2 335 698 B1**
(45) Date of publication and mention of the grant of the patent: **26.03.2014**
(21) Application number: 09811535.5
(22) Date of filing: 03.09.2009
(51) Int. Cl.: A61K 9/16, A61K 31/195, A61K 31/197, A61K 31/198, A61K 31/375, A61K 31/4415, A61K 9/20

(54) **PHARMACEUTICAL SOLID PREPARATION HAVING ACTIVE INGREDIENTS SEPARATED BY BOUNDARY THEREIN**
FESTE PHARMAZEUTISCHE ZUBEREITUNG MIT GRENZARTIG GETRENNTEN WIRKSTOFFEN
PRÉPARATION PHARMACEUTIQUE SOLIDE CONTENANT DES INGRÉDIENTS ACTIFS SÉPARÉS PAR UNE FRONTIÈRE INTERNE

(30) Priority: 05.09.2008 JP 2008227971
(43) Date of publication of application: 22.06.2011
(73) Proprietor: Daiichi Sankyo Healthcare Co., Ltd., Chuo-ku Tokyo 103-8541 (JP)
(72) Inventor: MATSUSHITA, Takeshi, Chuo-ku Tokyo 103-8541 (JP); MATSUBARA, Ken, Toyama-shi Toyama 939-8221 (JP); NARITA, Azusa, Toyama-shi Toyama 939-8221 (JP); MOCHIZUKI, Yusuke, Chuo-ku Tokyo 103-8541 (JP)
(74) Representative: Oldroyd, Richard Duncan
(86) International application number: PCT/JP2009/065390
(87) International publication number: WO 2010/027010

(56) References cited:
- EP-A1- 1 586 315
- WO-A1-2006/003965
- JP-A- 4 243 825
- JP-A- 2004 091 473
- JP-A- 2004 217 655
- JP-A- 2007 131 547
- DATABASE WPI Week 200578 Thomson Scientific, London, GB; AN 2005-763563 XP002654024, & JP 2005 314403 A (DAIICHI PHARM CO LTD) 10 November 2005 (2005-11-10)
- DATABASE WPI Week 198922 Thomson Scientific, London, GB; AN 1989-160819 XP002654025, & JP 1 102073 A (TAKEDA CHEM IND LTD) 19 April 1989 (1989-04-19)
- DATABASE WPI Week 200223 Thomson Scientific, London, GB; AN 2002-174320 XP002654026, & JP 2001 294524 A (TAISHO PHARM CO LTD) 23 October 2001 (2001-10-23)

## Description

### [Technical Field]

The present invention relates to a solid preparation for oral administration which is less likely to cause discoloration of the preparation, comprising tranexamic acid and ascorbic acid being separated by a boundary therebetween.

### [Background Art]

Tranexamic acid has an anti-bleeding effect (as an antiplasmin), an antiallergic effect, an antiinflammatory effect and the like, is widely used as ethical drugs and is incorporated also in OTC drugs. After it was reported in 1979 that when tranexamic acid was administered to a patient with chronic urticaria, chloasma which happened to occur at the same time in the same patient disappeared, tranexamic acid began to be prescribed for the treatment of chloasma (see, for example, Non-patent Document 1).

It has been known since long time ago that ascorbic acid suppresses the production of melanin pigment, and also that due to the action of degrading melanin pigment which has been already accumulated, ascorbic acid suppresses pigmentation. As for the OTC drugs, in the approved indications of preparations containing vitamin C as a base component, blots, freckles, pigmentation caused by sunburn or rash are included (see Non-patent Document 2).

Further, a preparation for treatment of pigmentation containing tranexamic acid and ascorbic acid has been disclosed, and is reported to show a superior degree of improvement of pigmentation than the case where tranexamic acid or ascorbic acid is administered as a single drug (see Patent Document 1).

Further, a skin whitening composition containing tranexamic acid, ascorbic acid and L-cysteine has been disclosed by the present inventors, and it has been known that pigmentation is further suppressed than the concomitant use of only tranexamic acid and ascorbic acid (see Patent Document 2).

It has not been known so far that there is an incompatibility between tranexamic acid and ascorbic acid.

### [Prior Art Documents]

### [Patent Documents]

[Patent Document 1] JP-A-4-243825
[Patent Document 2] JP-A-2004-217655

### [Non-patent Documents]

[Non-patent Document 1] Pharmacia Vol. 44, No. 5, 2008, pp. 437-442
[Non-patent Document 2] Ippan-yo Iyakuhin Seizo (Yunyu) Shonin Kijun (Approval Standards for Manufacturing (Import) of Non-Prescription Drugs), Jiho, 2008

### [Summary of the Invention]

### [Problem to BE Solved]

The present inventors actually produced, on a trial basis, a granule containing tranexamic acid and ascorbic acid disclosed in Example 1 of Patent Document 1 and recognized that there was a problem that the granule gradually changed its color (red discoloration) when it was subjected to a long term storage over time (in an accelerated test at a temperature of 40°C and a relative humidity of 75%).

Accordingly, an object of the invention is to provide a solid preparation for oral administration which contains tranexamic acid and ascorbic acid and is excellent in stability without causing discoloration or the like.

### [Means to Solve the Problem]

As a result of extensive studies on preparations by repeating trial and error to solve the above problems, the present inventors finally discovered facts as described below, and thus completed the invention.
1. Discoloration of a preparation can be prevented by producing the preparation by separately preparing:
   (a) a granule containing tranexamic acid; and
   (b) a granule containing ascorbic acid.
2. Further, in the case where "pyridoxine" is incorporated in the preparation described in 1, discoloration of the preparation can be prevented by incorporating L-cysteine in the pyridoxine-containing granule.
3. Further, in the case where "L-cysteine" is incorporated in the preparation described in 1, discoloration of the preparation can be prevented by incorporating pantothenic acid in the L-cystein-containing granule.
4. Further, in the case where "pyridoxine and pantothenic acid" are incorporated in the preparation described in 1, discoloration of the preparation can be prevented by incorporating L-cysteine in the granule that contains pyridoxine and pantothenic acid.

That is, the present invention is an invention shown below.
(1) A solid preparation for oral administration comprising, as active ingredients, tranexamic acid and ascorbic acid, wherein these active ingredients exist in the preparation being separated by a boundary therebetween.
(2) A solid preparation for oral administration comprising:
   (a) a component containing tranexamic acid as an active ingredient; and
   (b) a component containing ascorbic acid as an active ingredient,
      wherein components (a) and (b) exist being separated by a boundary therebetween.
(3) The solid preparation according to (2), wherein each of the components recited in (2) is in a granular form.
(4) The solid preparation according to (2) or (3), wherein either one or both of components (a) and (b) contain L-cysteine and pyridoxine.
(5) The solid preparation according to (2) or (3), wherein either one or both of components (a) and (b) contain L-cysteine and pantothenic acid.
(6) The solid preparation according to (2) or (3), wherein either one or both of components (a) and (b) contain L-cysteine, pantothenic acid and pyridoxine.
(7) The solid preparation for oral administration according to (4) or (6), wherein the pyridoxine is pyridoxine hydrochloride.
(8) The solid preparation for oral administration according to (5) or (6), wherein the pantothenic acid is calcium pantothenate.

### [Effect of the Invention]

The solid preparation for oral administration of the present invention is stable and useful because discoloration is prevented even if tranexamic acid and ascorbic acid are incorporated therein. Further, the solid preparation for oral administration of the present invention is also stable and useful because discoloration is prevented even if L-cysteine, pyridoxine or/and pantothenic acid is/are further incorporated therein.

### [Mode for Carrying Out the Invention]

The "pyridoxine" of the invention includes pyridoxine and a salt thereof, and is preferably pyridoxine hydrochloride.

The "pantothenic acid" of the invention includes pantothenic acid and a salt thereof, and is preferably calcium pantothenate.

The "solid preparation" of the invention includes a granule, a pill, a powder, a tablet, a capsule and the like listed in the Japanese Pharmacopoeia Fifteenth Edition.

The solid preparation for oral administration of the invention is preferably a granule, a powder, a capsule or a tablet.

Further, as a preferred embodiment of the granule or tablet of the invention, those coated with a sugar or a film are included. That is, a sugar-coated granule, a film-coated granule, a sugar-coated tablet, a film-coated tablet and the like are included.

In the case where the solid preparation for oral administration of the invention is a tablet, a multilayer tablet obtained by overlapping two layers or three or more layers of powders or granules with different compositions and compression-molding the resulting multilayer article is also included as a preferred embodiment.

The phrase "exist being separated by a boundary therebetween" as used herein refers to a state in which in the case where plural medicinal agents and the like exist in a solid preparation, these medicinal agents and the like exist in the same solid preparation without uniformly mixing with one another and without directly reacting with one another. For example, a solid preparation in which a substance which does not react with medicinal agents such as a binder or an excipient exists between the respective medicinal agents, or the respective medicinal agents are incorporated in different granules and exist in the preparation as plural granules is included.

Since medicinal agents "exist being separated by a boundary therebetween" in this manner, even if plural medicinal agents exist in the same solid preparation, the medicinal agents do not substantially come in contact with one another, thereby discoloration of the solid preparation can be prevented.

The examples of a method for producing the solid preparation of the invention include the following method.
a method comprising the steps of:
(1) separately preparing (a) a granule containing tranexamic acid and (b) a granule containing ascorbic acid; and
(2) in the case of a tablet, mixing the respective granules and then compression-molding the resulting mixture.

Tranexamic acid, ascorbic acid, calcium pantothenate and pyridoxine hydrochloride to be used in the invention are listed in the Japanese Pharmacopoeia Fifteenth Edition.

Further, L-cysteine is listed in Japanese Pharmaceutical Excipients 2003.

In the case where the solid preparation of the invention contains tranexamic acid, ascorbic acid, L-cysteine, pyridoxine hydrochloride and calcium pantothenate, the respective content ratios of ascorbic acid, L-cysteine, pyridoxine hydrochloride and calcium pantothenate based on 1 part by weight of tranexamic acid are as follows.

Preferred are 0.01 to 10 parts by weight, 0.01 to 10 parts by weight, 0.001 to 1 part by weight and 0.001 to 1 part by weight, respectively, more preferred are 0.1 to 2 parts by weight, 0.1 to 2 parts by weight, 0.001 to 0.5 part by weight and 0.01 to 0.5 part by weight, respectively.

### [Examples]

For illustrating the invention in more detail, Examples and Comparative Examples will be described below.

### 1. Preparation of Test Granules and Tablets

### (Comparative Example 1)

In a fluidized bed granulator, 96.2 g of tranexamic acid, 38.5 g of ascorbic acid and an appropriate amount of crystalline cellulose were applied and mixed. Then, an aqueous hydroxypropyl cellulose solution was sprayed, thereby a granule was obtained.

Talc was mixed with the granule, and a portion of the resulting granule was used as a test granule. The rest of the granule was mixed with crystalline cellulose, croscarmellose sodium and magnesium stearate, and the resulting mixture was tableted, thereby a tablet was obtained.

### (Comparative Example 2)

In a fluidized bed granulator, 96.2 g of tranexamic acid, 38.5 g of ascorbic acid, 30.8 g of L-cysteine, 0.8 g of pyridoxine hydrochloride and an appropriate amount of crystalline cellulose were applied and mixed, and then, an aqueous hydroxypropyl cellulose solution was sprayed, thereby a granule was obtained.

Talc was mixed with the granule, and a portion of the resulting granule was used as a test granule. The rest of the granule was mixed with crystalline cellulose, croscarmellose sodium and magnesium stearate, and the resulting mixture was tableted, thereby a tablet was obtained.

### (Comparative Example 3)

In a fluidized bed granulator, 750 mg of tranexamic acid, 300 mg of ascorbic acid, 240 mg of L-cysteine, 37 mg of calcium pantothenate, 6 mg of pyridoxine hydrochloride and an appropriate amount of crystalline cellulose were applied and mixed, and then, an aqueous hydroxypropyl cellulose solution was sprayed, thereby a granule was obtained.

Talc was mixed with the granule, and a portion of the resulting granule was used as a test granule. The rest of the granule was mixed with crystalline cellulose, croscarmellose sodium and magnesium stearate, and the resulting mixture was tableted, thereby a tablet was obtained.

### (Comparative Example 4)

In a fluidized bed granulator, 96.2 g of tranexamic acid, 38.5 g of ascorbic acid and an appropriate amount of crystalline cellulose were applied and mixed, and then, an aqueous hydroxypropyl cellulose solution was sprayed, thereby a granule 1 was obtained. Further, 30.8 g of L-cysteine, 4.7 g of calcium pantothenate, 0.8 g of pyridoxine hydrochloride and an appropriate amount of crystalline cellulose were mixed. Then, an aqueous hydroxypropyl cellulose solution was sprayed, thereby a granule 2 was obtained.

Talc was mixed with each of the granules 1 and 2, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Comparative Example 5)

In a fluidized bed granulator, 96.2 g of tranexamic acid, 30.8 g of L-cysteine and an appropriate amount of crystalline cellulose were applied and mixed. Then, an aqueous hydroxypropyl cellulose solution was sprayed, thereby a granule a was obtained. Further, 38.5 g of ascorbic acid, 4.7 g of calcium pantothenate, 0.8 g of pyridoxine hydrochloride and an appropriate amount of crystalline cellulose were mixed, and then, an aqueous hydroxypropyl cellulose solution was sprayed, thereby a granule b was obtained.

Talc was mixed with each of the granules a and b, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Comparative Example 6)

In a fluidized bed granulator, 96.2 g of tranexamic acid, 0.8 g of pyridoxine hydrochloride and an appropriate amount of crystalline cellulose were applied and mixed. Then, an aqueous hydroxypropyl cellulose solution was sprayed, thereby a granule a was obtained. Further, 38.5 g of ascorbic acid, 30.8 g of L-cysteine, 4.7 g of calcium pantothenate and an appropriate amount of crystalline cellulose were mixed. Then, an aqueous hydroxypropyl cellulose solution was sprayed, thereby a granule b was obtained.

Talc was mixed with each of the granules a and b, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Comparative Example 7)

In a fluidized bed granulator, 96.2 g of tranexamic acid, 4.7 g of calcium pantothenate, 0.8 g of pyridoxine hydrochloride and an appropriate amount of crystalline cellulose were applied and mixed. Then, an aqueous hydroxypropyl cellulose solution was sprayed, thereby a granule a was obtained. Further, 30.8 g of L-cysteine, 38.5 g of ascorbic acid and an appropriate amount of crystalline cellulose were mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Comparative Example 8)

In a fluidized bed granulator, 96. 2 g of tranexamic acid, 30.8 g of L-cysteine, 4.7 g of calcium pantothenate and an appropriate amount of crystalline cellulose were applied and mixed. Then, an aqueous hydroxypropyl cellulose solution was sprayed, thereby a granule a was obtained. Further, 38.5 g of ascorbic acid, 0.8 g of pyridoxine hydrochloride and an appropriate amount of crystalline cellulose were mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Example 1)

In a fluidized bed granulator, 96.2 g of tranexamic acid and an appropriate amount of crystalline cellulose were applied and mixed. Then, an aqueous hydroxypropyl cellulose solution was sprayed, thereby a granule a was obtained. Further, 38.5 g of ascorbic acid and an appropriate amount of crystalline cellulose were applied and mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Example 2)

In a fluidized bed granulator, 96.2 g of tranexamic acid and an appropriate amount of crystalline cellulose were applied and mixed, and then, an aqueous hydroxypropyl cellulose solution was sprayed, thereby a granule a was obtained. Further, 38.5 g of ascorbic acid, 30.8 g of L-cysteine, 0.8 g of pyridoxine hydrochloride and an appropriate amount of crystalline cellulose were applied and mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Example 3)

In a fluidized bed granulator, 96.2 g of tranexamic acid and an appropriate amount of crystalline cellulose were applied and mixed. Then, an aqueous hydroxypropyl cellulose solution was sprayed, thereby a granule a was obtained. Further, 38.5 g of ascorbic acid, 30.8 g of L-cysteine, 4.7 g of calcium pantothenate, 0.8 g of pyridoxine hydrochloride and an appropriate amount of crystalline cellulose were mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Example 4)

In a fluidized bed granulator, 2403.8 g of tranexamic acid, 769.2 g of L-cysteine, 118.6 g of calcium pantothenate, 19.2 g of pyridoxine hydrochloride and an appropriate amount of crystalline cellulose were applied and mixed. Then, an aqueous hydroxypropyl cellulose solution was sprayed, thereby a granule a was obtained. Further, 961.5 g of ascorbic acid and an appropriate amount of crystalline cellulose were mixed, and then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### (Example 5)

In a fluidized bed granulator, 2403.8 g of tranexamic acid, 118. 6 g of calcium pantothenate and an appropriate amount of crystalline cellulose were applied and mixed. Then, an aqueous hydroxypropyl cellulose solution was sprayed, thereby a granule a was obtained. Further, 961.5 g of ascorbic acid, 769.2 g of L-cysteine, 19.2 g of pyridoxine hydrochloride and an appropriate amount of crystalline cellulose were mixed. Then, an aqueous hydroxypropyl cellulose solution was sprayed, thereby a granule b was obtained.

Talc was mixed with each of the granules, and a portion of each of the resulting granules was used as a test granule. The rest of the granules were mixed with crystalline cellulose, croscarmellose sodium and magnesium stearate, and the resulting mixture was tableted, thereby a tablet was obtained.

### 2. Test Method

The granules of Examples 1 to 5 and Comparative Examples 1 to 8, and the tablets obtained by tableting the granules were left at 50°C for 4 days (only the granule of Comparative Example 3 was left at room temperature for 2 weeks), and discoloration of the respective granules and tablets was visually observed using those stored in a refrigerator as controls. The visual evaluation was performed according to the following criteria.

### (Evaluation Criteria)

A: no discoloration
B: slight discoloration
C: some discoloration
D: significant discoloration

### 3. Test Results

From the results shown in Table 1, it was found that discoloration of the preparation is caused when tranexamic acid and ascorbic acid are not separated by a boundary, and on the other hand, discoloration of the preparation is significantly prevented when tranexamic acid and ascorbic acid are separated by a boundary.

From the results shown in Tables 2 and 3, discoloration can be caused when pyridoxine is further incorporated in the case where tranexamic acid and ascorbic acid are separated by a boundary.

It was found that, even in such a case, discoloration can be significantly prevented by incorporating L-cysteine along with pyridoxine in (a) a granule containing tranexamic acid, or by incorporating L-cysteine along with pyridoxine in (b) a granule containing ascorbic acid.

Further, discoloration can be significantly prevented by incorporating L-cysteine along with pyridoxine in both of (a) a granule containing tranexamic acid and (b) a granule containing ascorbic acid.

From the results shown in Table 4, discoloration can be caused when L-cysteine is further incorporated in the case where tranexamic acid and ascorbic acid are separated by a boundary.

It was found that, even in such a case, discoloration can be significantly prevented by incorporating pantothenic acid along with L-cysteine in (a) a granule containing tranexamic acid, or by incorporating pantothenic acid along with L-cysteine in (b) a granule containing ascorbic acid.

From the results shown in Table 5, it was found that discoloration is caused also when pyridoxine and pantothenic acid are allowed to exist in the same granule in the case where tranexamic acid and ascorbic acid are separated by a boundary.

It was found that, even in such a case, discoloration can be significantly prevented by incorporating L-cysteine along with pyridoxine and pantothenic acid in (a) a granule containing tranexamic acid, or by incorporating L-cysteine along with pyridoxine and pantothenic acid in (b) a granule containing ascorbic acid.

By combining the results shown in Tables 2 to 5, it was found that when another medicinal agent is incorporated in addition to tranexamic acid and ascorbic acid being separated by a boundary, the followings should be performed in order to prevent discoloration.
(1) In the case where pyridoxine is further incorporated, pyridoxine should be incorporated along with L-cysteine in the same component.
(2) In the case where L-cysteine is further incorporated, L-cysteine should be incorporated along with pantothenic acid in the same component.
(3) In the case where pyridoxine and pantothenic acid are further incorporated, pyridoxine and pantothenic acid should be incorporated along with L-cysteine in the same component.

**(Table 1)**

| Component | | Comparative Example 1 | Comparative Example 2 | Comparative Example 3 | Comparative Example 4 | | Example 1 | |
|---|---|---|---|---|---|---|---|---|
| | | Single granule | Single granule | Single granule | Granule 1 | Granule 2 | Granule a | Granule b |
| Tranexamic acid | | ○ | ○ | ○ | ○ | | ○ | |
| Ascorbic acid | | ○ | ○ | ○ | ○ | | | ○ |
| L-cysteine | | | ○ | ○ | | ○ | | |
| Pyridoxine hydrochloric acid | | | ○ | ○ | | ○ | | |
| Ca pantothenate | | | | ○ | | ○ | | |
| Granule | 50°C, 4 days | D | C | (C) | D | C | A | B |
| Tablet | 50°C, 4 days | D | C | C | D | | B | |

In Table 1, pyridoxine hydrochloric acid denotes pyridoxine hydrochloride, and Ca pantothenate denotes calcium pantothenate.

**(Table 2)**

| Component | | Comparative Example 5 | | Comparative Example 6 | | Comparative Example 7 | | Comparative Example 8 | |
|---|---|---|---|---|---|---|---|---|---|
| | | Granule a | Granule b | Granule a | Granule b | Granule a | Granule b | Granule a | Granule b |
| Tranexamic acid | | ○ | | ○ | | ○ | | ○ | |
| Ascorbic acid | | | ○ | | ○ | | ○ | | ○ |
| L-cysteine | | ○ | | | ○ | | ○ | ○ | |
| Pyridoxine hydrochloric acid | | | ○ | ○ | | ○ | | | ○ |
| Ca pantothenate | | | ○ | | ○ | ○ | | ○ | |
| Granule | 50°C, 4 days | B | D | D | A | D | C | A | D |
| Tablet | 50°C, 4 days | D | | D | | D | | C | |

In Table 2, pyridoxine hydrochloric acid denotes pyridoxine hydrochloride, and Ca pantothenate denotes calcium pantothenate.

**(Table 3)**

| Component | | Example 2 | | Example 3 | | Example 4 | | Example 5 | |
|---|---|---|---|---|---|---|---|---|---|
| | | Granule a | Granule b | Granule a | Granule b | Granule a | Granule b | Granule a | Granule b |
| Tranexamic acid | | ○ | | ○ | | ○ | | ○ | |
| Ascorbic acid | | | ○ | | ○ | | ○ | | ○ |
| L-cysteine | | | ○ | | ○ | ○ | | | ○ |
| Pyridoxine hydrochloric acid | | | ○ | | ○ | ○ | | | ○ |
| Ca jantothenate | | | | | ○ | ○ | | ○ | |
| Granule | 50°C, 4 days | A | B | A | A | A | B | A | B |
| Tablet | 50°C, 4 days | B | | A | | A | | A | |

In Table 3, pyridoxine hydrochloric acid denotes pyridoxine hydrochloride, and Ca pantothenate denotes calcium pantothenate.

**(Table 4)**

| Component | | | | | |
|---|---|---|---|---|---|
| | | Granule a of Comparative Example 5 | Granule a of Comparative Example 8 | Granule b of Comparative Example 7 | Granule b of Comparative Example 6 |
| Tranexamic acid | | ○ | ○ | | |
| Ascorbic acid | | | | ○ | ○ |
| L-cysteine | | ○ | ○ | ○ | ○ |
| Pyridoxine hydrochloric acid | | | | | |
| Ca pantothenate | | | ○ | | ○ |
| Granule | 50°C, 4 days | B | A | C | A |

In Table 4, pyridoxine hydrochloric acid denotes pyridoxine hydrochloride, and Ca pantothenate denotes calcium pantothenate.

**(Table 5)**

| Component | | | | | | | |
|---|---|---|---|---|---|---|---|
| | | Single granule of Comparative Example 3 | Granule 2 of Comparative Example 4 | Granule b of Comparative Example 5 | Granule a of Comparative Example 7 | Granule b of Example 3 | Granule a of Example 4 |
| Tranexamic acid | | ○ | | | ○ | | ○ |
| Ascorbic acid | | ○ | | ○ | | ○ | |
| L-cysteine | | ○ | ○ | | | ○ | ○ |
| Pyridoxine hydrochloric acid | | ○ | ○ | ○ | ○ | ○ | ○ |
| Ca pantothenate | | ○ | ○ | ○ | ○ | ○ | ○ |
| Granule | 50°C, 4 days | (C) | C | D | D | A | A |

In Table 5, pyridoxine hydrochloric acid denotes pyridoxine hydrochloride, and Ca pantothenate denotes calcium pantothenate.

## Claims

1. A solid preparation for oral administration comprising:
(a) a component containing tranexamic acid as an active ingredient; and,
(b) a component containing ascorbic acid as an active ingredient,
wherein components (a) and (b) exist being separated by a boundary therebetween.

2. The solid preparation according to claim 1, wherein each of the components recited in claim 1 is in a granular form.

3. The solid preparation according to claim 1 or 2, wherein either one or both of components (a) and (b) contain L-cysteine and pyridoxine.

4. The solid preparation according to claim 1 or 2, wherein either one or both of components (a) and (b) contain L-cysteine and pantothenic acid.

5. The solid preparation according to claim 1 or 2, wherein either one or both of components (a) and (b) contain L-cysteine, pantothenic acid and pyridoxine.

6. The solid preparation for oral administration according to claim 3 or 5, wherein the pyridoxine is pyridoxine hydrochloride.

7. The solid preparation for oral administration according to claim 4 or 5, wherein the pantothenic acid is calcium pantothenate.

## Patentansprüche

1. Präparat in fester Form zur oralen Verabreichung, umfassend:
(a) eine Komponente, die Tranexamsäure als aktiven Bestandteil aufweist; und
(b) eine Komponente, die Ascorbinsäure als einen aktiven Bestandteil aufweist;
wobei die Komponenten (a) und (b) getrennt durch eine Begrenzung zwischen ihnen existieren.

2. Präparat in fester Form nach Anspruch 1, wobei jede der Komponenten aus Anspruch 1 in granularer Form gegeben ist.

3. Präparat in fester Form nach Anspruch 1 oder 2, wobei eine der Komponenten oder beide Komponenten (a) und (b) L-Cystein und Pyridoxin aufweisen.

4. Präparat in fester Form nach Anspruch 1 oder 2, wobei eine der Komponenten oder beide Komponenten (a) und (b) L-Cystein und Pantothensäure aufweisen.

5. Präparat in fester Form nach Anspruch 1 oder 2, wobei eine der Komponenten oder beide Komponenten (a) und (b) L-Cystein, Pantothensäure und Pyridoxin aufweisen.

6. Präparat in fester Form zur oralen Verabreichung nach Anspruch 3 oder 5, wobei es sich bei dem Pyridoxin um Pyridoxinhydrochlorid handelt.

7. Präparat in fester Form zur oralen Verabreichung nach Anspruch 4 oder 5, wobei es sich bei der Pantothensäure um Calciumpantothenat handelt.

## Revendications

1. Préparation solide pour administration orale comprenant :
(a) un composant contenant de l'acide tranexamique comme ingrédient actif ; et,
(b) un composant contenant de l'acide ascorbique comme ingrédient actif, dans laquelle les composants (a) et (b) sont séparés par une frontière.

2. Préparation solide selon la revendication 1, dans laquelle chacun des composants cités dans la revendication 1 est sous forme granulaire.

3. Préparation solide selon la revendication 1 ou 2, dans laquelle l'un ou les deux composants (a) et (b) contiennent de la L-cystéine et de la pyridoxine.

4. Préparation solide selon la revendication 1 ou 2, dans laquelle l'un ou les deux composants (a) et (b) contiennent de la L-cystéine et de l'acide pantothénique.

5. Préparation solide selon la revendication 1 ou 2, dans laquelle l'un ou les deux composants (a) et (b) contiennent de la L-cystéine, de l'acide pantothénique et de la pyridoxine.

6. Préparation solide pour administration orale selon la revendication 3 ou 5, dans laquelle la pyridoxine est du chlorhydrate de pyridoxine.

7. Préparation solide pour administration orale selon la revendication 4 ou 5, dans laquelle l'acide pantothénique est du pantothénate de calcium.
